# EUROPEAN PATENT APPLICATION

(11) **EP 3 363 798 A1**
(43) Date of publication of application: **22.08.2018**
(21) Application number: 17382083.8
(22) Date of filing: 20.02.2017
(51) Int. Cl.: C07D 403/12, A61K 31/506, A61P 1/00

(54) **AMORPHOUS FORM OF TELOTRISTAT ETIPRATE**

(71) Applicant: Esteve Química, S.A., 08024 Barcelona (ES)
(72) Inventor: WINTER, Stephen Benedict David, E-08024 Barcelona (ES); BERENGUER MAIMÓ, Ramón, E-08024 Barcelona (ES)
(74) Representative: ABG Patentes, S.L.

(57) **Abstract**

The present invention relates to amorphous forms of telotristat etiprate, to processes for preparing them, to pharmaceutical compositions comprising them, and to their medical use for the treatment of serotonin-mediated diseases or disorders.

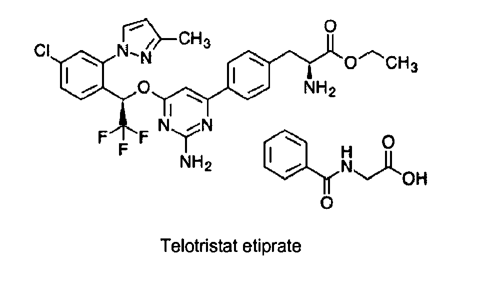

## Description

### Field of the Invention

The present invention relates to amorphous forms of telotristat etiprate, to processes for preparing them, to pharmaceutical compositions comprising them, and to their medical use for the treatment of serotonin-mediated diseases or disorders.

### Background of the Invention

In pharmaceutical development, amorphous and disordered crystalline materials are typically encountered in excipients, e.g., PVP, microcrystalline cellulose or magnesium stearate. However, when it comes to the active ingredient, new drug candidates generally enter the pharmaceutical development process in a crystalline state as this can ensure a high level of active ingredient purity and stability. On the contrary, whenever amorphous active ingredient material is present there is usually significant concern since, relative to the crystalline state, the amorphous state is less thermodynamically stable and exhibits greater chemical instability, in practice leading to great difficulty in processing, storage, and use of the pharmaceutical product containing the amorphous form. Also, since the amorphous state is metastable relative to the crystalline state, unexpected crystallization during storage is not uncommon, leading to macroscopic changes in specific surface area, flow, and a lack of control over pharmaceutical properties such as bioavailability.

Patent application WO 2009/042733 A1 discloses telotristat ethyl, i.e. (S)-ethyl 2-amino-3-(4-(2-amino-6-((R)-1-(4-chloro-2-(3-methyl-1H-pyrazol-1-yl)phenyl)-2,2,2-trifluoroethoxy)pyrimidin-4-yl)phenyl)propanoate. In line with the above, the invention disclosed therein is directed to solid crystalline forms of the compound or of its salts.

Furthermore, (S)-ethyl 2-amino-3-(4-(2-amino-6-((R)-1-(4-chloro-2-(3-methyl-1H-pyrazol-1-yl)phenyl)-2,2,2-trifluoroethoxy)pyrimidin-4-yl)phenyl)propanoate and crystalline salts thereof, in particular its crystalline hippurate salt, are known for their marked vulnerability to hydrolysis, which leads to major drawbacks and limitations when preparing and medically employing pharmaceutical compositions comprising these compounds. Patent application WO 2013/059146 A1 suggests overcoming this problem by formulating (S)-ethyl 2-amino-3-(4-(2-amino-6-((R)-1-(4-chloro-2-(3-methyl-1H-pyrazol-1-yl)phenyl)-2,2,2-trifluoroethoxy)pyrimidin-4-yl)phenyl)propanoate and crystalline salts thereof, in particular its crystalline hippurate salt, as specific dosage forms, in particular along with very specific excipients.

The present inventors have however now found that amorphous forms of a particular salt of (S)-ethyl 2-amino-3-(4-(2-amino-6-((R)-1-(4-chloro-2-(3-methyl-1 H-pyrazol-1-yl)phenyl)-2,2,2-trifluoroethoxy)pyrimidin-4-yl)phenyl)propanoate (telotristat ethyl), namely of the hippurate salt (also known as telotristat etiprate), are surprisingly highly stable, which accompanied to increased solubility and bioavailability typical of amorphous forms, unexpectedly renders their use as a pharmaceutical active ingredient particularly attractive.

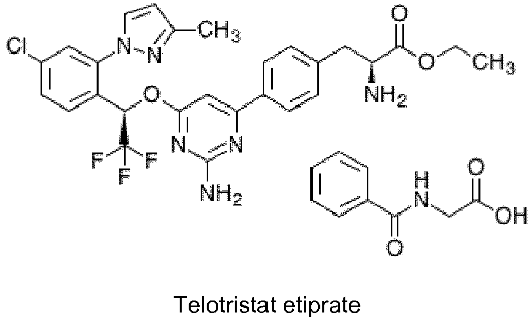

### Summary of the Invention

Thus, in a first aspect, the present invention is directed to an amorphous form of the hippurate salt of (S)-ethyl 2-amino-3-(4-(2-amino-6-((R)-1-(4-chloro-2-(3-methyl-1H-pyrazol-1-yl)phenyl)-2,2,2-trifluoroethoxy)pyrimidin-4-yl)phenyl)propanoate.

In another aspect, the invention relates to processes for preparing an amorphous form of the hippurate salt of (S)-ethyl 2-amino-3-(4-(2-amino-6-((R)-1-(4-chloro-2-(3-methyl-1 H-pyrazol-1-yl)phenyl)-2,2,2-trifluoroethoxy)pyrimidin-4-yl)phenyl)propanoate.

In a further aspect, the invention relates to pharmaceutical compositions comprising an amorphous form of the hippurate salt of (S)-ethyl 2-amino-3-(4-(2-amino-6-((R)-1-(4-chloro-2-(3-methyl-1H-pyrazol-1-yl)phenyl)-2,2,2-trifluoroethoxy)pyrimidin-4-yl)phenyl)propanoate.

In yet another aspect, the invention is directed to an amorphous form of the hippurate salt of (S)-ethyl 2-amino-3-(4-(2-amino-6-((R)-1-(4-chloro-2-(3-methyl-1H-pyrazol-1-yl)phenyl)-2,2,2-trifluoroethoxy)pyrimidin-4-yl)phenyl)propanoate for use in medicine, and more particularly for use in preventing or treating a serotonin-mediated disease or disorder.

### Brief description of the drawings

Figure 1. X-ray diffraction diffractogram (XRD) of the amorphous form of the hippurate salt of (S)-ethyl 2-amino-3-(4-(2-amino-6-((R)-1-(4-chloro-2-(3-methyl-1H-pyrazol-1-yl)phenyl)-2,2,2-trifluoroethoxy)pyrimidin-4-yl)phenyl)propanoate obtained by the method described in Example 1.
Figure 2. X-ray diffraction diffractogram (XRD) of the amorphous form of the hippurate salt of (S)-ethyl 2-amino-3-(4-(2-amino-6-((R)-1-(4-chloro-2-(3-methyl-1H-pyrazol-1-yl)phenyl)-2,2,2-trifluoroethoxy)pyrimidin-4-yl)phenyl)propanoate obtained by the method described in Example 2.
Figure 3: X-ray diffraction diffractogram (XRD) of the amorphous form of the hippurate salt of (S)-ethyl 2-amino-3-(4-(2-amino-6-((R)-1-(4-chloro-2-(3-methyl-1H-pyrazol-1-yl)phenyl)-2,2,2-trifluoroethoxy)pyrimidin-4-yl)phenyl)propanoate obtained by the method described in Example 3.
Figure 4: X-ray diffraction diffractogram (XRD) of the crystalline form of the hippurate salt of (S)-ethyl 2-amino-3-(4-(2-amino-6-((R)-1-(4-chloro-2-(3-methyl-1H-pyrazol-1-yl)phenyl)-2,2,2-trifluoroethoxy)pyrimidin-4-yl)phenyl)propanoate prepared according to WO 2009/042733 A1 (Example 6.9).

### Detailed Description of the Invention

The first aspect of the present invention is directed to an amorphous form of the hippurate salt of (S)-ethyl 2-amino-3-(4-(2-amino-6-((R)-1-(4-chloro-2-(3-methyl-1H-pyrazol-1-yl)phenyl)-2,2,2-trifluoroethoxy)pyrimidin-4-yl)phenyl)propanoate. The hippurate salt of (S)-ethyl 2-amino-3-(4-(2-amino-6-((R)-1-(4-chloro-2-(3-methyl-1H-pyrazol-1-yl)phenyl)-2,2,2-trifluoroethoxy)pyrimidin-4-yl)phenyl)propanoate will hereinafter be referred to as telotristat etiprate.

In particular, the invention is directed to an amorphous form of telotristat etiprate defined by an X-ray diffraction diffractogram comprising a diffraction halo (broad band). More particularly, the halo ranges to about 33 degrees in 2θ. More particularly, the halo ranges from less than 16 to about 33 degrees in 2θ. More particularly, the halo peaks (i.e. reaches a count maximum) at about 20 degrees in 2θ.

As used herein, the term "about" means a slight variation of the value specified, preferably within 25% of the value specified.

The invention is also directed to an amorphous form of telotristat etiprate provided in the form of a dispersion, defined by an X-ray diffraction diffractogram comprising two diffraction halos.

More particularly, the first halo ranges from about 5 degrees in 2θ. More particularly, the first halo ranges from about 5 to beyond 16 degrees in 2θ, the end of the range not being observable due to superimposition with the second halo. More particularly, the first halo peaks at about 11.5 to 13.5 degrees in 2θ.

More particularly, the second halo ranges to about 33 degrees in 2θ. More particularly, the second halo ranges from less than 16 to about 33 degrees in 2θ, the beginning of the range not being observable due to superimposition with the first halo. More particularly, the second halo peaks at about 20 degrees in 2θ.

In any of the above embodiments, the diffractogram consists essentially of the stated pattern. More preferably, the diffractogram consists of the stated pattern.

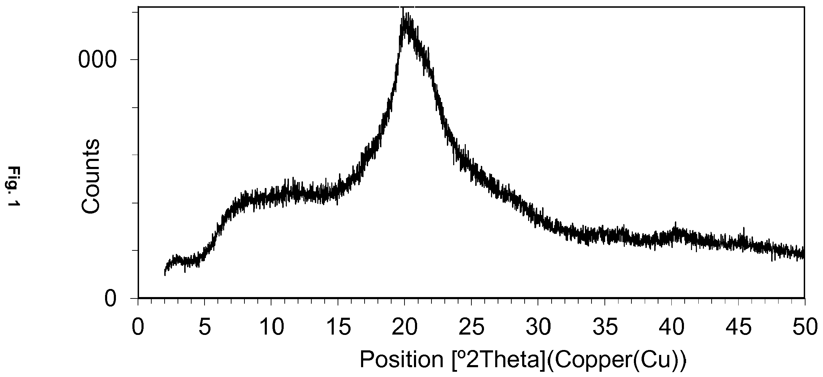

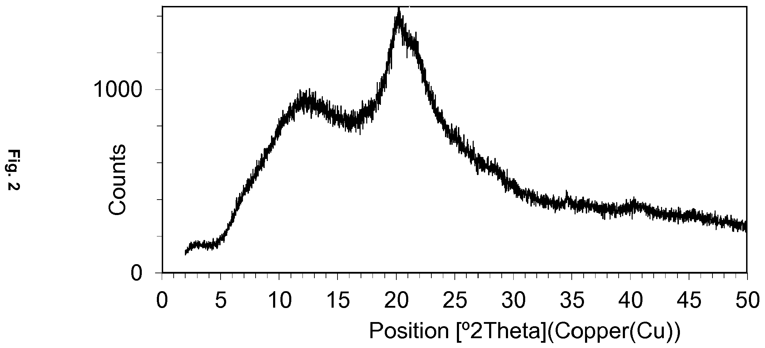

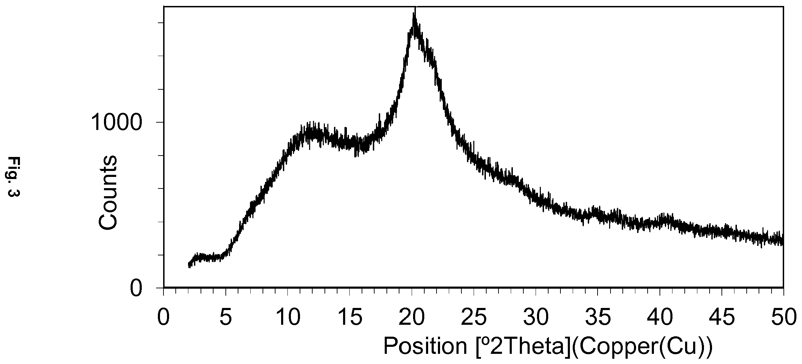

More particularly, the invention is directed to an amorphous form of telotristat etiprate defined by an X-ray diffraction diffractogram showing halos at degrees as shown in Figures 1, 2 or 3. In a particular embodiment, the halos are at degrees as shown in Figure 1. In another particular embodiment, the halos are at degrees as shown in Figure 2. In yet another particular embodiment, the halos are at degrees as shown in Figure 3.

As those skilled in the art are well aware, the relative intensities of peaks in an X-ray diffraction diffractogram can vary depending on how the sample is prepared and how the data is collected.

More particularly, the invention is directed to an amorphous form of telotristat etiprate defined by an X-ray diffraction diffractogram substantially in accordance with Figures 1, 2 or 3. In a particular embodiment, the X-ray diffraction diffractogram is substantially in accordance with Figure 1. In another particular embodiment, the X-ray diffraction diffractogram is substantially in accordance with Figure 2. In yet another particular embodiment, the X-ray diffraction diffractogram is substantially in accordance with Figure 3. In a preferred embodiment, an X-ray diffraction diffractogram substantially in accordance with a Figure is an X-ray diffraction diffractogram that may be non-identical to that depicted in the stated Figure, but that falls within the limits of experimental error, when considered by one of ordinary skill in the art.

More particularly, the invention is directed to an amorphous form of telotristat etiprate defined by an X-ray diffraction diffractogram as shown in Figures 1, 2 or 3. In a particular embodiment, the X-ray diffraction diffractogram is as shown in Figure 1. In another particular embodiment, the X-ray diffraction diffractogram is as shown in Figure 2. In yet another particular embodiment, the X-ray diffraction diffractogram is as shown in Figure 3.

No peaks assignable to crystalline forms are observable in the spectra of the above embodiments. The patterns described are those observable in the angular range of 0 to 50 degrees.

The spectra of the above embodiments were acquired on a powder diffraction system using Cu_{Kα1}-radiation in transmission geometry (Wavelength: 1.54 Å).

In a preferred embodiment, the amorphous form of telotristat etiprate is a solid form.

This invention encompasses mixtures, preferably solid mixtures, of both an amorphous form of telotristat etiprate as described herein, and a crystalline form of telotristat etiprate. Preferably, the amorphous form of telotristat etiprate is present in an amount of at least 50, at least 75, at least 80, at least 85, at least 90, at least 95 or at least 99 wt. % with respect to the combined weight of the amorphous form of telotristat etiprate and the crystalline form of telotristat etiprate. Preferably, the amorphous form of telotristat etiprate is present in an amount of from 50 to 99, from 75 to 99, from 80 to 99, from 85 to 99, from 90 to 99, from 95 to 99 wt. % with respect to the combined weight of the amorphous form of telotristat etiprate and the crystalline form of telotristat etiprate. Preferably, the amorphous form of telotristat etiprate is present in an amount of from 50 to 95, from 75 to 95, from 80 to 95, from 85 to 95, from 90 to 95 wt. % with respect to the combined weight of the amorphous form of telotristat etiprate and the crystalline form of telotristat etiprate. If more than one crystalline form of telotristat etiprate is present, the stated weight percentages refer to the amount of amorphous form of telotristat etiprate with respect to the combined weight of the amorphous form of telotristat etiprate and all crystalline forms of telotristat etiprate present in the mixture.

In a preferred embodiment, the amorphous form of telotristat etiprate is provided along with a polymeric material, in the form of a solid dispersion. The invention thus also refers to solid dispersions comprising an amorphous form of telotristat etiprate as described in any of the above embodiments and a polymeric material.

Preferably, the polymeric material should be pharmaceutically acceptable. Pharmaceutically acceptable means that the polymeric material is tolerated physiologically, normally meaning that it is not toxic when used for the treatment of a disease, disorder or condition in humans or animals.

Preferably, the polymeric material is a vinyl polymer or copolymer, or a cellulose-based polymer or copolymer.

A vinyl polymer or copolymer is a polymer or copolymer derived from vinyl monomers, the monomers being unsubstituted or substituted once or more times, and wherein each substituent is independently selected from alkyl, aryl, halogen, ester, amide, carboxylic acid, -OH and -CN. "Alkyl" refers to a straight or branched hydrocarbon chain radical consisting of 1 to 6 carbon atoms, containing no unsaturation, and which is attached to the rest of the monomer by a single bond, e.g., methyl, ethyl, or proyl, preferably methyl. "Aryl" refers to single and double aromatic ring radicals containing from 6 to 10 carbon ring atoms, such as phenyl or naphthyl, preferably phenyl. "Halogen" refers to bromo, chloro, iodo or fluoro, preferably chloro. "Ester" is an - OC(O)R group, wherein R is alkyl as previously defined. "Amide" is an -NC(O)R group, wherein R is alkyl as previously defined.

Preferably, the polymeric material is a poly (vinyl pyrrolidone) polymer or copolymer. The copolymer comprises poly (vinyl pyrrolidone) and a repeating unit selected from the above mentioned vinyl monomers. Preferably, the poly (vinyl pyrrolidone) copolymer is a poly(vinyl pyrrolidone)-poly(vinyl ester) copolymer, preferably a poly(vinyl pyrrolidone)-poly(vinyl acetate) copolymer. More preferably, the polymeric material is a poly (vinyl pyrrolidone) polymer. Poly (vinyl pyrrolidone) polymers are commercially available from Ashland® under the Plasdone® or Polyplasdone® series.

In a particular embodiment, the polymeric material is a poly (acrylate ester) polymer or copolymer, a poly (methacrylate ester) polymer or copolymer, or a poly (acrylate ester)-poly (methacrylate ester) copolymer. The ester is preferably a methyl ester. Such polymers are commercially available from EVONIK® under the EUDAGRIT® series.

In a particular embodiment, the polymeric material is a cellulose-based polymer or copolymer. In any of the above embodiments, the cellulose-based polymer or copolymer is preferably a hydroxypropyl methylcellulose polymer or copolymer, preferably a hydroxypropyl methylcellulose polymer.

More particularly, the dispersion is defined by an X-ray diffraction diffractogram showing halos at degrees as shown in Figures 2 or 3. In a particular embodiment, the halos are at degrees as shown in Figure 2. In yet another particular embodiment, the halos are at degrees as shown in Figure 3.

In a particular embodiment, the dispersion is defined by an X-ray diffraction diffractogram substantially in accordance with that shown in Figures 2 or 3. In a more particular embodiment, the dispersion is defined by an X-ray diffraction diffractogram substantially in accordance with that shown in Figure 2. In another more particular embodiment, the dispersion is defined by an X-ray diffraction diffractogram substantially in accordance with that shown in Figure 3.

### Preparation processes

In another aspect the invention also provides processes for preparing an amorphous form of telotristat etiprate, which comprise dissolving telotristat etiprate in a suitable solvent to form a solution (also referred to herein as the spray solution); and spray-drying the spray solution.

The form of telotristat etiprate which is preferably dissolved is the crystalline form. This form can be obtained as described for example in patent application WO 2009/042733 A1 (Example 6.9) or may be commercially obtained for instance from AdooQ Bioscience (Ref: A14299; CAS No. 11137608-69-5).

Suitable solvents are organic solvents in which telotristat etiprate is soluble. The solvent is preferably water or a polar organic solvent, in particular water or a polar protic or polar aprotic solvent, or a mixture thereof. Particular examples of suitable solvents are water, acetonitrile, C₁-C₆ aliphatic alcohols (e.g. ^{t}butanol, n-butanol, isopropanol, n-propanol, ethanol, methanol), ethyl acetate, tetrahydrofuran, dichloromethane, acetone, dimethylformamide, dimethyl sulfoxide, acetic acid, formic acid, or a mixture thereof. Preferably, the solvent is selected from water, acetonitrile, C₁-C₆ aliphatic alcohols, and mixtures thereof. Preferably, the solvent is selected from water, acetonitrile, and mixtures thereof.

In a preferred embodiment, the solvent is a mixture of water and any of the above mentioned organic solvents. More preferably, the solvent is a mixture of water and acetonitrile, or of water and a C₁-C₆ aliphatic alcohol. Even more preferably, the solvent is a mixture of water and acetonitrile. Preferably, the ratio of water to organic solvent in any of the previous embodiments is from 2:1 to 1:2 (volume/volume), preferably about 1:1, more preferably 1:1.

In an embodiment, the concentration of telotristat etiprate in the spray solution is comprised in the range 1-30% wt/v (weight/volume, i.e. g/mL x 100), preferably 1-15% wt/v, even more preferably, 2-10% wt/v, most preferably 3-5% wt/v.

The term spray-drying is used conventionally and broadly refers to processes involving breaking up liquid mixtures into small droplets (atomization) and rapidly removing solvent from the droplets in a container where there is a strong driving force for evaporation of solvent.

In a preferred embodiment, spray-drying involves:
a) Providing a spray drier apparatus having an atomizer connected to a drying chamber, the drying chamber having an inlet and an outlet;
b) Spraying the spray solution through the atomizer into the drying chamber to form fine (atomized) droplets whilst flowing a drying gas through the inlet and into the drying chamber, where the solvent evaporates from the droplets, and solid amorphous drug particles are formed.

Typically, the flow rate and inlet temperature of the drying gas is usually such that the droplets solidify in a given time, usually in less than about twenty seconds.

After formation of the solid amorphous drug particles, the exhaust drying gas (formed by the now cooled drying gas and evaporated solvent) exit the drying chamber out of an outlet at the bottom of the drying chamber. The solid amorphous dispersion particles may be separated from the exhaust gas by means of a cyclone or other collection device.

In a preferred embodiment, the drying gas is an inert gas, such as nitrogen.

In an embodiment, the drying gas inlet temperature is between 50 and 150°C, preferably between 90 and 110°C. More preferably the inlet temperature is between 99 and 103°C.

In an embodiment, the drying gas outlet temperature is between 30 and 90°C, preferably between 50 and 70°C. More preferably the outlet temperature is between 55 and 65°C.

In a preferred embodiment, the spray solution is filtered through a syringe prior to spray drying.

The above process and process embodiments are also suitable for preparing the dispersions described above. These dispersions comprise a polymeric material, as was described above. The polymeric material is dissolved along with the crystalline telotristat etiprate in a suitable solvent to form the spray solution.

When the dispersion is to be prepared, a particularly preferred concentration of telotristat etiprate plus polymeric material in the spray solution is comprised in the range 1-30% wt/v, preferably 5-15% wt/v, even more preferably, 9-13% wt/v.

It should be noted that all the above preferred and particular process embodiments may me combined to arrive at further concrete embodiments.

The present invention is further directed to an amorphous form of telotristat etiprate, or a dispersion, obtainable by the above described process of preparation or by any of its embodiments.

### Medical uses and compositions

In another aspect the present invention also refers to the medical use of an amorphous form of telotristat etiprate as described in any of the above embodiments, or of a dispersion as described in any of the above embodiments, and more particularly to the medical use for inhibiting tryptophan hydroxylase (TPH), and preferably the TPH1 isoform thereof.

Thus, the present invention provides an amorphous form of telotristat etiprate, or a dispersion as described above, for use in medicine. More particularly, the present invention provides an amorphous form of telotristat etiprate, or a dispersion as described above, for use in preventing or treating a serotonin-mediated disease or disorder.

Alternatively, the present invention provides the use of an amorphous form of telotristat etiprate, or of a dispersion as described above, in the preparation of a medicament for the prevention or treatment of a serotonin-mediated disease or disorder.

Alternatively, the present invention provides a method for preventing or treating a serotonin-mediated disease or disorder, comprising administering to a subject in need of such treatment an amorphous form of telotristat etiprate, or a dispersion as described above.

Preferably, the serotonin-mediated disease or disorder is selected from abdominal pain (e.g., associated with medullary carcinoma of the thyroid), anxiety, carcinoid syndrome, celiac disease, constipation (e.g. , constipation having an iatrogenic cause, and idiopathic constipation), Crohn's disease, depression, diabetes, diarrhea (e.g., bile acid diarrhea, enterotoxin-induced secretory diarrhea, diarrhea having an iatrogenic cause, idiopathic diarrhea (e.g. , idiopathic secretory diarrhea), and traveler's diarrhea), emesis, functional abdominal pain, functional dyspepsia, irritable bowel syndrome (IBS), lactose intolerance, Multiple endocrine neoplasia (MEN) types I and II, Ogilvie's syndrome, Pancreatic Cholera Syndrome, pancreatic insufficiency, pheochromacytoma, scleroderma, somatization disorder, and Zollinger-Ellison Syndrome.

Preferably, the serotonin-mediated disease or disorder is carcinoid syndrome.

It is to be understood that, when medically used, the amorphous form of telotristat etiprate, or the dispersion, is used in therapeutically effective amounts. The physician will determine the dosage of the amorphous form of telotristat etiprate, or of the dispersion, which is therapeutically effective according to the type of pharmaceutical formulation or form of administration chosen. Pharmaceutical formulations and modes of administration are described further below. Other factors that will influence the chosen dosage are the age of the patient, and the type of disease or disorder being treated.

As used herein, the term "treatment" or derivations thereof include the eradication, removal, reversion, alleviation, modification, or control of a serotonin-mediated disease or disorder.

As used herein, the term "prevention" or derivations thereof refer to the avoiding or minimizing of the onset or development of a serotonin-mediated disease or disorder.

In still another aspect the present invention provides pharmaceutical compositions which comprise one or more pharmaceutically acceptable excipients. Pharmaceutically acceptable means that the excipient is tolerated physiologically, normally meaning that it is not toxic when used for the treatment of a disease, disorder or condition in humans or animals.

The term "excipient" refers to components of a drug compound other than the active ingredient. They preferably include a "carrier, adjuvant and/or vehicle". Carriers are forms to which substances are incorporated to improve the delivery and the effectiveness of drugs. Drug carriers are used in drug-delivery systems such as the controlled-release technology to prolong *in vivo* drug actions, decrease drug metabolism, and reduce drug toxicity. Carriers are also used in designs to increase the effectiveness of drug delivery to the target sites of pharmacological actions. Adjuvant is a substance added to a drug product formulation that affects the action of the active ingredient in a predictable way. Vehicle is an excipient or a substance, preferably without therapeutic action, used as a medium to give bulk for the administration of medicines. Such pharmaceutical carriers, adjuvants or vehicles can be sterile liquids, such as water and oils, including those of petroleum, animal, vegetable or synthetic origin, such as peanut oil, soybean oil, mineral oil, sesame oil and the like, excipients, disgregants, wetting agents or diluents.

The pharmaceutical compositions disclosed herein can be formulated for oral, topical, mucosal (e.g., nasal, pulmonary, sublingual, vaginal, buccal, or rectal), parenteral (e.g., subcutaneous, intravenous, bolus injection, intramuscular, or intraarterial), or transdermal administration to a patient. Examples of dosage forms include, but are not limited to: tablets; caplets; capsules, such as soft elastic gelatin capsules; cachets; troches; lozenges; dispersions; suppositories; ointments; cataplasms (poultices); pastes; powders; dressings; creams; plasters; solutions; patches; aerosols (e.g., nasal sprays or inhalers); gels; liquid dosage forms suitable for oral or mucosal administration to a patient, including suspensions (e.g., aqueous or non-aqueous liquid suspensions, oil-in-water emulsions, or a water-in-oil liquid emulsions), solutions, and elixirs; liquid dosage forms suitable for parenteral administration to a patient; and sterile solids (e.g., crystalline or amorphous solids) that can be reconstituted to provide liquid dosage forms suitable for parenteral administration to a patient.

In a preferred embodiment the pharmaceutical compositions are in oral form, either solid or liquid. Suitable dose forms for oral administration may be tablets, pills, caplets, gel caps, chewing gums, capsules, granules, drops, syrups or solutions and may contain conventional excipients known in the art such as binding agents, for example syrup, acacia, gelatin, sorbitol, tragacanth, or polyvinylpyrrolidone; fillers, for example lactose, sugar, maize starch, calcium phosphate, sorbitol or glycine; tabletting lubricants, for example magnesium stearate; disintegrants, for example starch, polyvinylpyrrolidone, sodium starch glycolate or microcrystalline cellulose; or pharmaceutically acceptable wetting agents such as sodium lauryl sulfate.

The pharmaceutical composition formulation should suit the mode of administration. For example, oral administration may require enteric coatings to protect the active ingredient from degradation within the gastrointestinal tract. In another example, the active ingredient may be administered in a liposomal formulation to shield it from degradative enzymes, facilitate transport in circulatory system, and/or effect delivery across cell membranes to intracellular sites.

A dosage form used in the acute treatment of a disease may contain larger amounts of active ingredient than a dosage form used in the chronic treatment of the same disease. Similarly, a parenteral dosage form may contain smaller amounts of active ingredient than an oral dosage form used to treat the same disease.

The present invention is also directed to the medical use of pharmaceutical compositions as described in any of the above embodiments, wherein the medical use is also as described in any of the above medical use embodiments.

In a preferred embodiment, the pharmaceutical composition is administered at a dose of amorphous form of telotristat etiprate of from 50 to 600 mg. Preferred doses are 150 mg, 250 mg, 350 mg and 500 mg, most preferably 250 or 500 mg. Preferably, these doses are administered one, twice, thrice, or four times daily, particularly preferably three times a day. In particular, the dose is administered for at least 4 weeks, at least 6 weeks, at least 8 weeks, at least 10 weeks, or preferably at least 12 weeks or for about 12 weeks. In a particularly preferred embodiment, a progressively increasing dose is administered to the patient until reaching a maximum dose, which is then maintained for the remainder of the treatment. In a particular embodiment, the dose progression is as follows: 150 mg, 250 mg, 350 mg and finally 500 mg. The most preferred mode of administration is oral administration, in particular as described above.

The subject which is treated is a human or animal subject, preferably a human subject.

It should be noted that all the above preferred and particular embodiments may me combined to arrive at further concrete embodiments.

The following examples are merely illustrative of certain embodiments of the invention and cannot be considered as restricting it in any way.

### Examples

### Example 1: Preparation of amorphous form of (S)-ethyl 2-amino-3-(-4-(2-amino-6-((R)-1-(4-chloro-2-(3-methyl-1H-pyrazol-1-yl)phenyl)-2,2, 2-trifluoroethoxy)pyrimidin-4-yl)phenyl)propanoate hippurate

1 g of crystalline (S)-ethyl 2-amino-3-(-4-(2-amino-6-((R)-1-(4-chloro-2-(3-methyl-1H-pyrazol-1-yl)phenyl)-2,2,2-trifluoroethoxy)pyrimidin-4-yl)phenyl)propanoate hippurate was prepared as described in WO 2009/042733 A1 (Example 6.9) and then dissolved in 25mL of a 1:1 mixture of acetonitrile/water (v/v) in a 50 mL round bottom flask with magnetic stirring at ambient temperature. The resulting solution was filtered with a syringe filter and spray-dried using a Büchi B-290 mini spray dryer with the following conditions: inlet temperature: 100°C, outlet temperature: 56°C, feed rate: 9%, spray gas flow: 40 mm, to give the amorphous form of Telotristat etiprate.

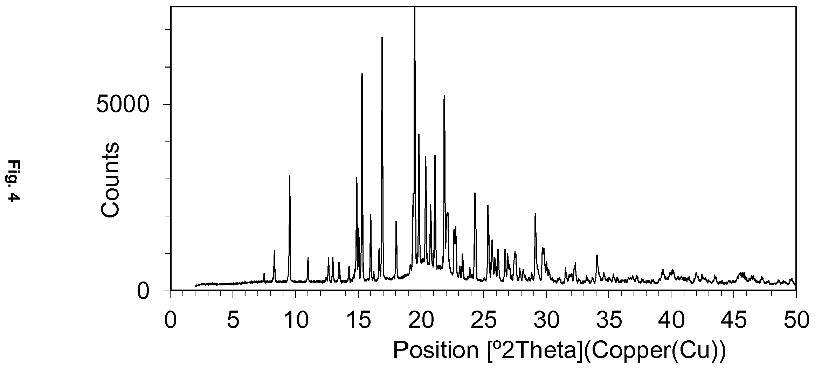

Figure 1 is an X-ray powder diffraction pattern of the obtained amorphous form of (S)-ethyl 2-amino-3-(-4-(2-amino-6-((R)-1-(4-chloro-2-(3-methyl-1H-pyrazol-1-yl)phenyl)-2,2,2-trifluoroethoxy)pyrimidin-4-yl)phenyl)propanoate hippurate. Figure 4 is an X-ray diffraction pattern of the corresponding crystalline starting material prepared according to WO 2009/042733 A1 (Example 6.9).

A sample of the amorphous product in a vial was stored under conditions of 40°C, 75%RH and reanalyzed after 1 and 4 months by X-ray powder diffraction (XRD), and shown to be amorphous with a diffractogram the same as at time zero.

### Example 2: Preparation of amorphous form of (S)-ethyl 2-amino-3-(4-(2-amino-6-((R)-1-(4-chloro-2-(3-methyl-1H-pyrazol-1-yl) phen yl)-2, 2, 2-trifluoroethoxy)pyrimidin-4-yl)phenyl)propanoate hippurate as a solid dispersion with Plasdone^{®}

1 g of crystalline (S)-ethyl 2-amino-3-(-4-(2-amino-6-((R)-1-(4-chloro-2-(3-methyl-1H-pyrazol-1-yl)phenyl)-2,2,2-trifluoroethoxy)pyrimidin-4-yl)phenyl)propanoate hippurate prepared according to WO 2009/042733 A1 (Example 6.9) and 5 g of Plasdone™ were dissolved in 50 mL of a mixture of acetonitrile/H₂O (1:1). The resulting solution was filtered with a syringe filter and spray-dried using a Büchi B-290 mini spray dryer with the following conditions: inlet temperature: 102°C, outlet temperature: 60°C, feed rate: 9%, spray gas flow: 40 mm, to give the amorphous form.

Figure 2 is an X-ray powder diffraction pattern of the amorphous form of (S)-ethyl 2-amino-3-(-4-(2-amino-6-((R)-1-(4-chloro-2-(3-methyl-1H-pyrazol-1-yl)phenyl)-2,2,2-trifluoroethoxy)pyrimidin-4-yl)phenyl)propanoate hippurate obtained as a solid dispersion with Plasdone™.

A sample of the product in a vial was stored under conditions of 40°C, 75%RH and reanalyzed after 1 and 4 months by XRD, and shown to be amorphous with a diffractogram the same as at time zero.

### Example 3: Preparation of amorphous form of (S)-ethyl 2-amino-3-(4-(2-amino-6-((R)-1-(4-chloro-2-(3-methyl-1H-pyrazol-1-yl) phen yl)-2, 2, 2-trifluoroethoxy)pyrimidin-4-yl)phenyl)propanoate hippurate as a solid dispersion with PVP

0.5 g of crystalline (S)-ethyl 2-amino-3-(-4-(2-amino-6-((R)-1-(4-chloro-2-(3-methyl-1H-pyrazol-1-yl)phenyl)-2,2,2-trifluoroethoxy)pyrimidin-4-yl)phenyl)propanoate hippurate prepared according to WO 2009/042733 A1 (Example 6.9) and 2.5 g of PVP were dissolved in 30 mL of a mixture of acetonitrile/H₂O (1:1). The resulting solution was filtered with a syringe filter and spray-dried using a Büchi B-290 mini spray dryer with the following conditions: inlet temperature: 100°C, outlet temperature: 63°C, feed rate: 9%, spray gas flow: 40 mm, to give the amorphous form.

Figure 3 is an X-ray powder diffraction pattern of the amorphous form of (S)-ethyl 2-amino-3-(-4-(2-amino-6-((R)-1-(4-chloro-2-(3-methyl-1H-pyrazol-1-yl)phenyl)-2,2,2-trifluoroethoxy)pyrimidin-4-yl)phenyl)propanoate hippurate obtained as a solid dispersion with PVP.

A sample of the product in a vial was stored under conditions of 40°C, 75%RH and reanalyzed after 1 and 4 months by XRD, and shown to be amorphous with a diffractogram the same as at time zero.

## Claims

1. An amorphous form of the hippurate salt of (S)-ethyl 2-amino-3-(4-(2-amino-6-((R)-1-(4-chloro-2-(3-methyl-1H-pyrazol-1-yl)phenyl)-2,2,2-trifluoroethoxy)pyrimidin-4-yl)phenyl)propanoate.

2. Amorphous form according to claim 1, **characterized by** an X-ray powder diffraction diffractogram comprising a halo which peaks at about 20 degrees in 2θ; wherein the 2θ values are obtained using copper radiation (Cu_{Kα1} 1.54 Å).

3. Amorphous form according to claims 1 or 2, **characterised by** an X-ray powder diffraction diffractogram substantially in accordance with that shown in Figure 1.

4. A solid dispersion comprising:
- an amorphous form of the hippurate salt of (S)-ethyl 2-amino-3-(4-(2-amino-6-((R)-1-(4-chloro-2-(3-methyl-1 H-pyrazol-1-yl)phenyl)-2,2,2-trifluoroethoxy)pyrimidin-4-yl)phenyl)propanoate as defined in any of claims 1 to 3; and
- a polymeric material.

5. Solid dispersion according to claim 4, wherein the polymeric material is selected from a poly (vinyl pyrrolidone) polymer or copolymer.

6. Solid dispersion according to claim 5, **characterized by** an X-ray powder diffraction diffractogram substantially in accordance with that shown in Figure 2 or Figure 3.

7. Process for the preparation of an amorphous form of the hippurate salt of (S)-ethyl 2-amino-3-(4-(2-amino-6-((R)-1-(4-chloro-2-(3-methyl-1H-pyrazol-1-yl)phenyl)-2,2,2-trifluoroethoxy)pyrimidin-4-yl)phenyl)propanoate as defined in any of claims 1 to 3, comprising the steps of:
a) Dissolving crystalline hippurate salt of (S)-ethyl 2-amino-3-(4-(2-amino-6-((R)-1-(4-chloro-2-(3-methyl-1H-pyrazol-1-yl)phenyl)-2,2,2-trifluoroethoxy)pyrimidin-4-yl)phenyl)propanoate in a solvent to form a solution;
b) Spray-drying the solution obtained in step a).

8. Process for the preparation of a dispersion as defined in any of claims 4 to 6, comprising the steps of:
a) Dissolving crystalline hippurate salt of (S)-ethyl 2-amino-3-(4-(2-amino-6-((R)-1-(4-chloro-2-(3-methyl-1H-pyrazol-1-yl)phenyl)-2,2,2-trifluoroethoxy)pyrimidin-4-yl)phenyl)propanoate, and a polymeric material, in a solvent to form a solution;
b) Spray-drying the solution obtained in step a).

9. Process according to claim 7 or 8, wherein the solvent is water, a polar organic solvent, or a mixture thereof, and wherein the organic solvent is preferably acetonitrile.

10. Process according to claim 9, wherein the solvent is a mixture of acetonitrile and water.

11. Pharmaceutical composition comprising:
- an amorphous form of the hippurate salt of (S)-ethyl 2-amino-3-(4-(2-amino-6-((R)-1-(4-chloro-2-(3-methyl-1 H-pyrazol-1-yl)phenyl)-2,2,2-trifluoroethoxy)pyrimidin-4-yl)phenyl)propanoate as defined in any of claims 1 to 3, or a dispersion as defined in any of claims 4 to 6; and
- a pharmaceutically acceptable excipient.

12. An amorphous form of the hippurate salt of (S)-ethyl 2-amino-3-(4-(2-amino-6-((R)-1-(4-chloro-2-(3-methyl-1H-pyrazol-1-yl)phenyl)-2,2,2-trifluoroethoxy)pyrimidin-4-yl)phenyl)propanoate as defined in any of claims 1 to 3, or a dispersion as defined in any of claims 4 to 6, for use in medicine.

13. An amorphous form of the hippurate salt of (S)-ethyl 2-amino-3-(4-(2-amino-6-((R)-1-(4-chloro-2-(3-methyl-1H-pyrazol-1-yl)phenyl)-2,2,2-trifluoroethoxy)pyrimidin-4-yl)phenyl)propanoate as defined in any of claims 1 to 3, or a dispersion as defined in any of claims 4 to 6, for use in the treatment of a serotonin-mediate disease or disorder.

14. An amorphous form of the hippurate salt of (S)-ethyl 2-amino-3-(4-(2-amino-6-((R)-1-(4-chloro-2-(3-methyl-1H-pyrazol-1-yl)phenyl)-2,2,2-trifluoroethoxy)pyrimidin-4-yl)phenyl)propanoate as defined in any of claims 1 to 3, or a dispersion as defined in any of claims 4 to 6, for use in the treatment of abdominal pain, anxiety, carcinoid syndrome, celiac disease, constipation, Crohn's disease, depression, diabetes, diarrhea, emesis, functional dyspepsia, irritable bowel syndrome, lactose intolerance, multiple endocrine neoplasia types I and II, Ogilvie's syndrome, pancreatic cholera syndrome, exocrine pancreatic insufficiency, pheochromocytoma, scleroderma, somatization disorder, and Zollinger-Ellison syndrome.

15. An amorphous form of the hippurate salt of (S)-ethyl 2-amino-3-(4-(2-amino-6-((R)-1-(4-chloro-2-(3-methyl-1H-pyrazol-1-yl)phenyl)-2,2,2-trifluoroethoxy)pyrimidin-4-yl)phenyl)propanoate as defined in any of claims 1 to 3, or a dispersion as defined in any of claims 4 to 6, for use in the treatment of carcinoid syndrome.
